# EUROPEAN PATENT SPECIFICATION

(11) **EP 4 292 640 B1**
(45) Date of publication and mention of the grant of the patent: **01.04.2026**
(21) Application number: 22752634.0
(22) Date of filing: 01.02.2022
(51) Int. Cl.: A61N 1/04, A61N 1/36, A61B 5/252, A61B 5/256, A61B 5/266

(54) **ELECTRODE MEMBER, HEAD ORTHOSIS, BODY ORTHOSIS, AND WEAR SET**
ELEKTRODENELEMENT, KOPFORTHESE, KÖRPERORTHESE UND VERSCHLEISSSET
ÉLÉMENT D'ÉLECTRODE, ORTHÈSE DE TÊTE, ORTHÈSE DE CORPS ET ENSEMBLE À PORTER

(30) Priority: 12.02.2021 JP 2021021263
(43) Date of publication of application: 20.12.2023
(73) Proprietor: Nakatsugawa, Shigekazu, Tokyo, 134-0083 (JP)
(72) Inventor: Nakatsugawa, Shigekazu, Tokyo, 134-0083 (JP)
(74) Representative: Dehns
(86) International application number: PCT/JP2022/003835
(87) International publication number: WO 2022/172806

(56) References cited:
- CN-A- 107 049 302
- JP-A- 2008 149 016
- JP-A- 2013 081 691
- JP-A- S57 160 438
- JP-A- S58 116 338
- JP-U- H 033 304
- JP-U- H0 339 008
- US-A- 4 693 711
- US-A1- 2011 054 288

## Description

### Technical Field

The present invention relates to an electrode member, a head orthosis, a body orthosis, and a wear set.

### Background Art

In recent years, the situation surrounding human beings is changing drastically, such as climate extremes due to environmental destruction caused by global warming, widening of disparities in all aspects due to the global economy, the possibility of war due to the intensification of conflict between the United States and China, and fear of death, particularly in the elderly and people with chronic diseases, due to the global spread of the new coronavirus. The disparity extends not only to economic power, nutrition, and education, but also to exercise, information acquisition, processing capability, and the like. In other words, it is no exaggeration to say that we are in the midst of a whirlwind of unprecedented upheaval. Under such circumstances, it is quite difficult for people to maintain good sleep sufficient in both quality and quantity, which is important for maintaining the mental calmness and physical health.

The life (daily activities) of people consists of about 34 to 50% in length of social activities outside of home, about 17 to 33% in length of home life, the remaining about 33% in sleep time, and is regulated by the body clock. It has been found that sleep, during which the memory is fixed, also plays a major role in maintenance of visceral function, and is essential for improving productivity. In a sense, activities other than sleep time are supported by, for example, the mind and the physical environment, that is, nutrition, exercise, sleep, lifestyle, memory, purpose of life, and the like, and conversely, the quality and quantity of sleep depend on the physical or psychological fatigue, stress, psychological state, other social activities, nutrition, exercise, lifestyle, and the like.

In addition, it is also known that sleep has large individual differences such as a change in body temperature, a balance of a heat release amount (sweating ability, etc.) together with a calorific value (exercise amount, meal contents, etc.), and is greatly affected by a climate of a living area, that is, an environment such as temperature, humidity, sound, and light. Regarding individual differences, large differences are generated in a wide range of areas such as individual body composition, in particular, muscle mass related to calorific value, cardiovascular system and nervous system related to skeleton, heat dissipation, and sweating, degree of development and aging of internal organs, combination of lifestyle diseases, balance between immunity and endocrine system, and quality, amount and contents of work.

Furthermore, in a case where the sleep time itself is shorter than 5 hours, in a case where the nap is 30 minutes or more, and in a case of sleep apnea syndrome or the like, it is known that the risk of sleep disorder and lifestyle diseases such as diabetes is high, and thus, the importance of the modulation mechanism of the autonomic nerve related to the biological clock is known, and it is suggested that the importance of ensuring a deep sleep time that continues nasal respiration that can make the parasympathetic nerve dominant. In this sense as well, it can be said that the know-how to realize good sleep including the sleep apnea syndrome treatment method (therapy for upper-airway restoration and maintenance: hereinafter referred to as "TURM") is essential in this era in which it is difficult to avoid the anxiety of living with-corona.

On the other hand, for reliable good sleep and falling asleep again, effects of acupuncture treatment including general massage have been known from long ago. In addition, in an uneasy psychological state such as lack of exercise, excessive eating, and high stress, and an increasing shift work and irregular work, waking up during sleep and early awakening frequently occur, and it is often difficult to fall asleep again. However, in recent years where the coronavirus infection continues, head massage or the like essentially needs a practitioner and is not realistic to be applied to sleep every night. Therefore, there is a demand for a methodology by which the user himself/herself produces an effect of falling asleep or re-falling asleep with the parasympathetic nerve dominance that can be relaxed similarly to the acupuncture treatment from an excitation activity state with the sympathetic nerve dominance.

As for not only sleep apnea syndrome (SAS) but also an overnight polygraph test (hereinafter referred to as "PSG test") known as a final test related to sleep, in our country, health insurance treatment fees have been significantly reduced since last spring, which incurs a large deficit against the required labor, and therefore facilities that stop the PSG test have been increased. As a result, it is socially difficult to diagnose SAS for which early detection and early treatment are desired, and it is expected that the PSG test will be strongly demanded to be carried out without labor cost and consumables cost.

In the PSG test, the following configurations are considered.
1) Electroencephalogram Frontal (F3, F4 as defined in the international 10-20 system): useful in determining delta waves appearing in deep sleep.
   Central (C3, C4 as defined in the international 10-20 system): optimal for observation of aneurysm wave or parietal sharp wave, sleep spindle wave, K compound and α wave as an index of awakening.
   Occipital (O1, O2 as defined in the international 10-20 system): to ensure determination of wakefulness (observing α waves).
2) Electrooculogram (derivation of changes in potential due to eye movement) important for differentiating stage R (REM sleep). Sleep onset can be predicted from gradual eye movement.
3) Jaw electromyogram of the jaw important for differentiating between wakefulness and REM sleep.
4) Electrocardiogram: to record heart rate, heart rate variability, arrhythmia, change in ST-T, and the like.
5) Arterial oxygen saturation (SpO2): important for determining apnea/hypopnea and determining the severity of sleep disordered breathing.
   SpO2 is measured using a pulse oximeter.
6) Respiration curve: important for classification of obstructive, central and mixed apnea and evaluation of hypopnea. The respiration curve requires measurement of air flow from the nostril and mouth and movement of the chest and abdomen.
   As the nose/mouth sensor, it is recommended to use a temperature sensor (thermistor method, thermocouple method) that senses a temperature change of the airflow, a detection sensor for hypopnea determination, and a pressure transducer (air pressure method) that detects an airflow change from a pressure difference as a detection sensor for apnea determination.
7) Snoring sound: a snoring sound is recorded with an acoustic sensor (such as a microphone).
8) Body position: In OSAS, there are an example in which the severity is reduced by sleeping in a lateral position and an example in which the severity is exacerbated by sleeping in a supine position, which are information useful for treatment.
9) Lower limb electromyogram: important for determining periodic limb movement (PLM). The tibialis anterior is used for the lower limb electromyogram.

As a PSG test device, for example, the technology of Patent Literature 1 has been proposed. Patent Literature 1 describes a device including a head orthosis for PSG test including an electroencephalogram electrode for acquiring an electroencephalogram of a user, an electrooculogram electrode for acquiring an electrooculogram of the user, and an oxygen saturation sensor for acquiring oxygen saturation of the user.

### Citation List

### Patent Literature

Patent Literature 1: JP 2013-81691 A

### Summary of Invention

### Technical Problem

However, in the PSG test in a case where a user is highly obese or sebum or the like adheres to the body surface such as scalp of the user, it is difficult to capture a weak current such as electroencephalogram separated by cerebrospinal fluid or skull. Therefore, in a case of using the PSG test device or the like described in Patent Literature 1, it is necessary to remove sebum or makeup of the user before the test, which takes time and effort. In addition, when the PSG test is carried out, apnea and hypopnea during sleep may be less likely to occur if the sleep is shallow, and thus it is considered that a device for inducing the user to sleep is also necessary.

In addition, there is a strong demand for a head orthosis, a body orthosis, and the like by which the user himself/herself produces an effect of falling asleep or re-falling asleep with the parasympathetic nerve dominance that can be relaxed similarly to the acupuncture treatment from an excitation activity state with the sympathetic nerve dominance.

The present invention has been made in view of the above circumstances, and an object of the present invention is to provide an electrode member, a head orthosis, a body orthosis, and a wear set that can reliably perform PSG test while suppressing the cost and the inspection labor.

### Solution to Problem

In order to solve the above problems, an electrode member of the present invention is an electrode member to be attached to a body surface of a user, and includes:
an adhesion part including a through hole near a center and capable of adhering to a body surface of a user;
an electrode inserted into the through hole of the adhesion part; and
a first retention part configured to retain a solution for dissolving sebum, in which a flow channel including a gap is formed between an inner peripheral surface of the through hole of the adhesion part and the electrode, and
the solution for dissolving sebum retained in the first retention part is able to flow out from one end side of the through hole through the flow channel.

A head orthosis of the present invention includes the electrode member and a head mounted member.

A body orthosis of the present invention includes the electrode member and a body mounted member.

The wear set of the present invention is characterized by including a head orthosis and a body orthosis.

### Advantageous Effects of Invention

With the electrode member, the head orthosis, the body orthosis, and the wear set of the present invention, it is possible to reliably perform the PSG test while reducing the cost and the time and effort for the test, and it is possible to reliably promote good sleep and re-falling asleep by the alternative therapeutic effect of the acupuncture treatment.

### Brief Description of Drawings

Fig. 1 is a longitudinal sectional view exemplifying a first embodiment of an electrode member of the present invention.
Fig. 2 is a schematic cross-sectional view exemplifying one mode of a method of attaching the electrode member illustrated in Fig. 1 to a body surface (head or the like) of a user.
Fig. 3 is a schematic cross-sectional view exemplifying one mode of a method of attaching the electrode member illustrated in Fig. 1 to a body surface (head or the like) of a user.
Fig. 4 is a schematic cross-sectional view exemplifying one mode of a method of attaching the electrode member illustrated in Fig. 1 to a body surface (head or the like) of a user.
Fig. 5 is a longitudinal sectional view exemplifying a second embodiment of an electrode member of the present invention.
Fig. 6 is a schematic cross-sectional view exemplifying one mode of a method of attaching an electrode member of the present invention to a body surface (head or the like) of a user according to a third embodiment.
Fig. 7 is a schematic cross-sectional view exemplifying one mode of a method of attaching an electrode member of the present invention to a body surface (head or the like) of a user according to the third embodiment.
Fig. 8 is a schematic cross-sectional view exemplifying one mode of a method of attaching an electrode member of the present invention to a body surface (head or the like) of a user according to the third embodiment.
Fig. 9 is a schematic cross-sectional view exemplifying one mode of a method of attaching an electrode member of the present invention to a body surface (head or the like) of a user according to the third embodiment.
Fig. 10 is a schematic cross-sectional view exemplifying one mode of a method of attaching an electrode member of the present invention to a body surface (head or the like) of a user according to the third embodiment.
Fig. 11 is a schematic cross-sectional view exemplifying one mode of a method of attaching an electrode member of the present invention to a body surface (head or the like) of a user according to a fourth embodiment.
Fig. 12 is a schematic cross-sectional view exemplifying one mode of a method of attaching an electrode member of the present invention to a body surface (head or the like) of a user according to the fourth embodiment.
Fig. 13 is a schematic cross-sectional view exemplifying one mode of a method of attaching an electrode member of the present invention to a body surface (head or the like) of a user according to the fourth embodiment.
Fig. 14 is a schematic cross-sectional view exemplifying one mode of a method of attaching an electrode member of the present invention to a body surface (head or the like) of a user according to the fourth embodiment.
Fig. 15 is a schematic cross-sectional view exemplifying one mode of a method of attaching an electrode member of the present invention to a body surface (head or the like) of a user according to the fourth embodiment.
Fig. 16(A) is a front view exemplifying an embodiment of a head orthosis of the present invention, and Fig. 16(B) is a cross-sectional view of the head orthosis.

### Description of Embodiments

Hereinafter, a first embodiment of an electrode member of the present invention will be described.

The electrode member of the present invention can be preferably used, for example, or for PSG test.

In recent years, falling asleep due to head massage has attracted attention, and it is considered that it is possible to solve sleep disorder, falling asleep disorder, or difficulty in falling asleep again after waking up by electric stimulation with the electrode members disposed on parasympathomes-related pressure points in not only the head and the neck, but also in the whole body. In addition, the electrode member of the present invention can acquire electroencephalogram (weak current) from the surface of the head or the like of a user also in the application of PSG test. In addition, the electrode member of the present invention can also be used for electroencephalogram measurement in consideration of localization diagnosis.

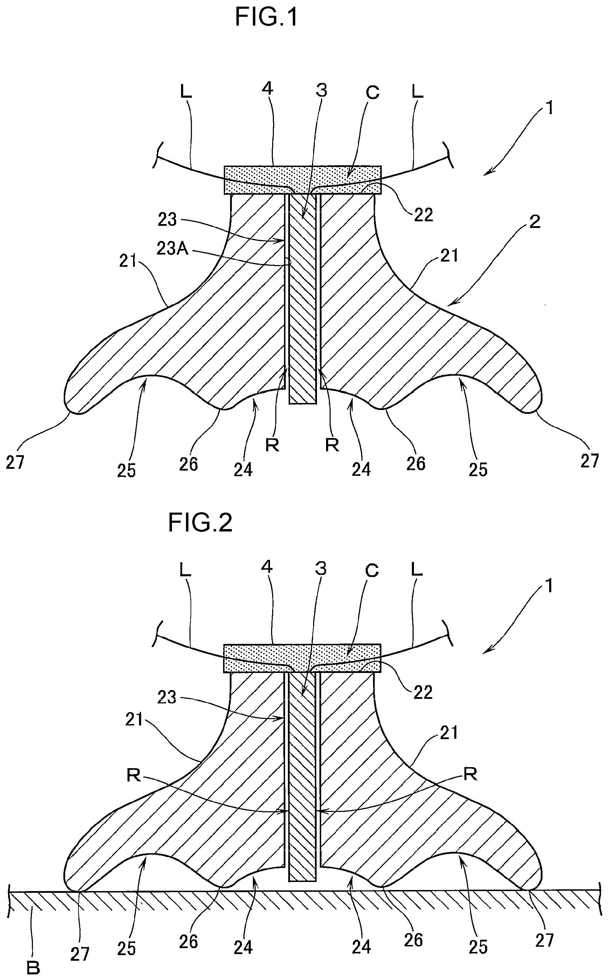

Fig. 1 is a longitudinal sectional view exemplifying an embodiment of an electrode member of the present invention.

The electrode member 1 includes an adhesion part 2, an electrode 3, and a first retention part 4A.

The adhesion part 2 can adhere to a body surface (including a head surface) of the user. Specifically, in this embodiment, the adhesion part 2 is formed with an inclined portion 21 that expands downward so as to be larger in diameter. In addition, a flat portion 22 is formed on the upper end side of the adhesion part 2, and a through hole 23 penetrating vertically is formed downward from the vicinity of the center of the flat portion 22.

A substantially circular first concave portion 24 formed around the opening end of the through hole 23 and concaved upward is formed on the lower end side of the adhesion part 2. Furthermore, annular second concave portion 25 concaved upward is formed around the first concave portion 24. Boundary portion 26 protruding downward is formed between the first concave portion 24 and the second concave portion 25. In addition, outer edge portion 27 protruding downward is formed outside the second concave portion 25. The second concave portion 25 is concaved more greatly upward than the first concave portion 24.

The material of the adhesion part 2 is not particularly limited, but for example, an elastic material such as silicone can be preferably exemplified.

The electrode 3 is inserted into the through hole 23 of the adhesion part 2. In this embodiment, an elongated rod-shaped electrode 3 is exemplified. As the electrode 3, a component capable of acquiring an electroencephalogram (EEG) of the user and composed from a conventionally known material (for example, copper or the like) can be used.

One end (lower end) of the electrode 3 is exposed in the first concave portion 24 of the adhesion part 2. The other end (upper end) of the electrode 3 is located in the vicinity of the flat portion 22 of the adhesion part 2, and can be connected to various measuring devices and the like via the conductive wire L.

A flow channel R defined with a gap between the inner peripheral surface 23A of the through hole 23 of the adhesion part 2 and the electrode 3 is formed. The flow channel R is designed to allow a liquid to move using a capillary phenomenon. The lower end of flow channel R is connected to the first concave portion 24 of the adhesion part 2.

In the mode for PSG test, a weak current on the body surface can be captured by the electrode 3. In addition, in the mode for massage, a current can be applied from the electrode 3 to the body surface to stimulate the body surface.

The first retention part 4A is hollow in which the solution for dissolving sebum C can be retained. The component of the solution for dissolving sebum C is not particularly limited, but preferably contains a surfactant, an alcohol, or a lipase, and the specific examples may include mixed liquids such as sodium laureth sulfate, ammonium polyoxyethylene lauryl ether sulfate, lauryl hydroxysulfobetaine, sodium laureth sulfate, stearyl alcohol, and triacylglyceride lipase. In a case where the user is not highly obese, the solution for dissolving sebum C is not necessarily required for a measurement site for an electromyogram, an electrocardiogram, and the like where the current is not weak as compared with the EEG, unlike the scalp.

In this embodiment, the first retention part 4A is provided on the flat portion 22 of the adhesion part 2 (the other end side of the through hole 23), and the solution for dissolving sebum C flows out from one end side of the through hole 23 through the flow channel R from the first retention part 4A and is supplied to the first concave portion 24.

Figs. 2 to 4 are schematic cross-sectional views each exemplifying one mode of a method of attaching the electrode member illustrated in Fig. 1 to a body surface (head or the like) of the user.

As illustrated in Fig. 2, when the electrode member 1 is attached to the body surface B, the electrode member 1 is disposed at a predetermined body surface B position, and initial pressing is performed from above the electrode member 1 toward the body surface B side. As a result, the adhesion part 2 is deformed and brought into close contact with the body surface B, the insides of the first concave portion 24 and the second concave portion 25 are brought into a negative pressure state, and the electrode member 1 adheres to the body surface B. In the mode illustrated in Fig. 2, the outer edge portion 27 of the adhesion part 2 is in contact with the body surface B. In addition, the solution for dissolving sebum C in the first retention part 4A falls down the flow channel R due to a capillary phenomenon and is easily sucked out to the first concave portion 24 side.

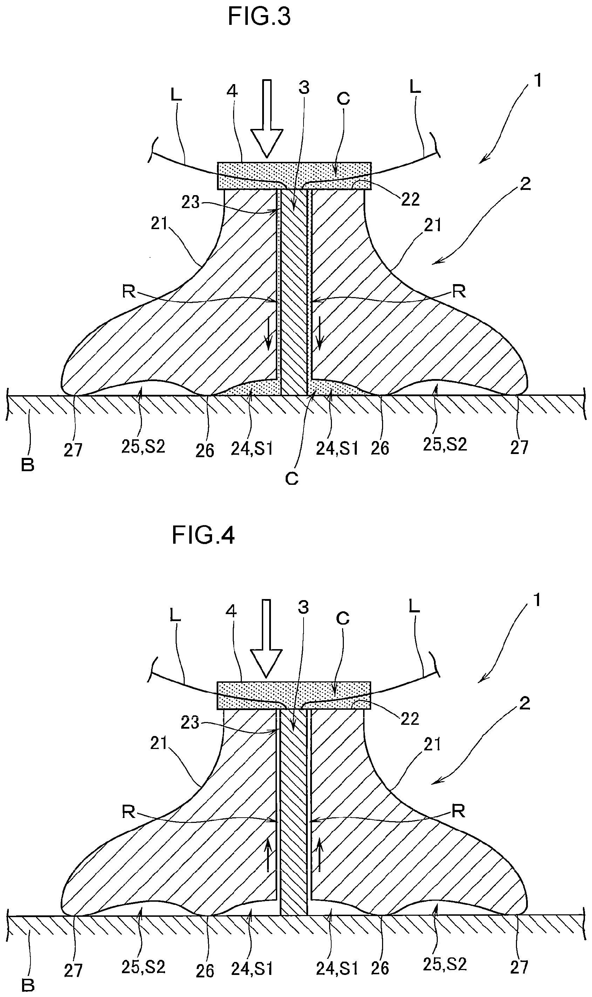

Next, as illustrated in Fig. 3, the second pressing is performed from the upper side of the electrode member 1 toward the body surface B side to deform the adhesion part 2, and thus the negative pressures inside the first concave portion 24 and the second concave portion 25 are increased. At this time, the boundary portion 26 and the outer edge portion 27 are in contact with the body surface B, the volume of the internal space of the first concave portion 24 and the volume of the internal space of the second concave portion 25 decrease, and the first space S1 and the second space S2 sealed with the body surface B are formed. Therefore, the first concave portion 24 (first space S1) is filled with the solution for dissolving sebum C that is supplied through the flow channel R and that is in contact with the body surface B. In addition, at this time, the lower end of the electrode 3 is designed to be in contact with the body surface B.

Then, the state in which the first concave portion 24 is filled with the solution for dissolving sebum C is maintained for about 5 to 30 minutes to dissolve the sebum on the body surface B.

Thereafter, as illustrated in Fig. 4, the third pressing is performed from the upper side of the electrode member 1 toward the body surface B side, the negative pressure in the first concave portion 24 is set to a positive pressure, and a part of the solution for dissolving sebum C is returned into the first retention part 4A by raising the flow channel R. As a result, after the sebum on the body surface B is dissolved, the electrode member 1 can be brought into close contact with and fixed to a predetermined position on the body surface B by the first concave portion 24 and the second concave portion 25. Therefore, in the mode for massage, it is possible to stimulate the body surface B by applying a current from the electrode 3 to the body surface B, at a predetermined position, and it is possible to relax the user and promote falling asleep. In addition, in the mode for PSG test, it is possible to reliably capture a weak current (such as EEG) from the electrode 3 to the body surface B, and it is possible to improve the inspection accuracy while suppressing the cost and the inspection labor. Furthermore, in the mode for massage, the electrode member 1 can enhance the massage effect by enabling low-frequency irradiation and ultrasonic irradiation. The frequency can be appropriately set in consideration of the target person, the massage effect, and the like.

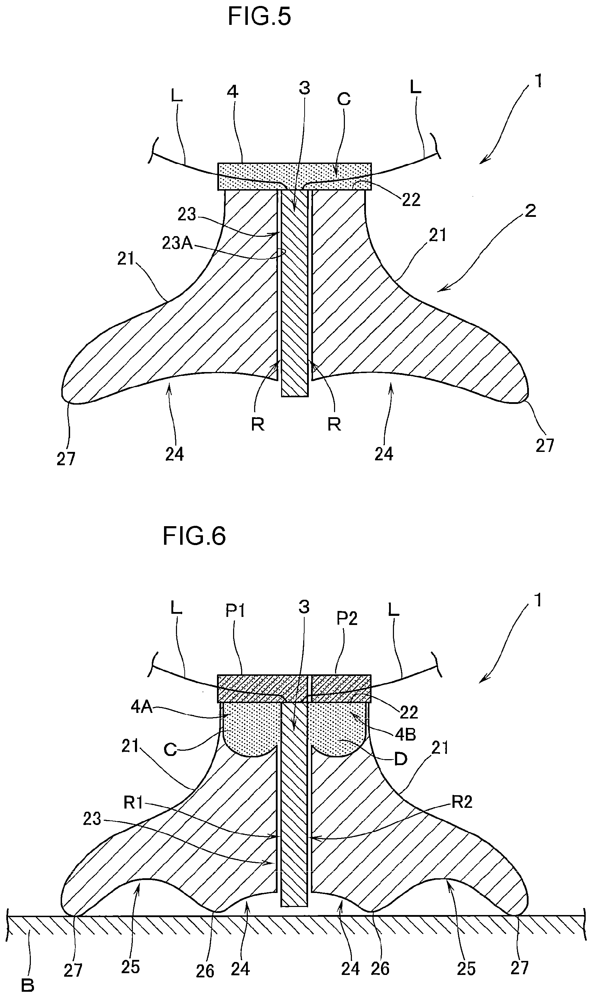

Next, a second embodiment of the electrode member of the present invention will be described with reference to Fig. 5. Portions common to those in the above embodiment are denoted by the same reference signs, and description thereof will be partially omitted.

In the electrode member 1 of this embodiment, a substantially circular first concave portion 24 concaved upward from the outer edge portion 27 toward the through hole 23 side is formed on the lower end side of the adhesion part 2. That is, in the electrode member 1 of this embodiment, the second concave portion in the above-described embodiment is not formed.

In the electrode member 1 of this embodiment, the electrode member 1 is disposed at a predetermined body surface B position, and the electrode member 1 is pressed from above toward the body surface side, so that the adhesion part 2 is deformed and brought into close contact with the body surface, the inside of the first concave portion 24 is brought into a negative pressure state, and the electrode member 1 adheres to the body surface. Then, the solution for dissolving sebum C supplied through the flow channel R fills between the body surface and the first concave portion 24, so that sebum on the body surface can be dissolved.

Also with the electrode member 1 of this embodiment, in the mode for massage, it is possible to stimulate the body surface B by applying a current from the electrode 3 to the body surface B, at a predetermined position, and it is possible to relax the user and promote falling asleep. In addition, in the mode for PSG test, it is possible to reliably capture a weak current (such as EEG) from the electrode 3 to the body surface B, and it is possible to improve the inspection accuracy while suppressing the cost and the inspection labor.

Furthermore, a third embodiment of the electrode member of the present invention will be described with reference to Figs. 6 to 10. Portions common to those of the first embodiment are denoted by the same reference signs, and description thereof will be partially omitted.

As illustrated in Fig. 6, the electrode member 1 of this embodiment includes a first retention part 4A in the upper side of the adhesion part 2 adjacent to the electrode 3. In the mode illustrated in Fig. 6, the first retention part 4A is formed on one side (left half in the drawing) of the adhesion part 2.

In addition, the electrode member 1 of this embodiment includes a second retention part 4B on the other side (right half in the drawing) of the adhesion part 2 adjacent to the first retention part 4A. The solution for dissolving sebum C is retained in the first retention part 4A, and the solution for dissolving sebum C is able to flow out from one end side of the through hole 23 through the flow channel (first flow channel R1).

The electrolyte gel D is retained in the second retention part 4B, and the electrolyte gel D is able to flow out from one end side of the through hole 23 through the flow channel (second flow channel R2). The component of the electrolyte gel D is not particularly limited, but it is preferable to contain a material that covers the narrowness of the contact area between the electrode 3 and the body surface B (prevents contact failure) and increases the conductivity.

Furthermore, the electrode member 1 includes a first pressing portion P1 on the upper side of the first retention part 4A and a second pressing portion P2 on the upper side of the second retention part 4B. The first pressing portion P1 and the second pressing portion P2 are divided and adjacent to each other, and can be operated independently.

When the electrode member 1 of this embodiment is attached to the body surface B, the electrode member 1 is disposed at a predetermined position on the body surface B.

Next, the electrode member 1 is pushed from above toward the body surface B side (first pressing). The first pressing portion P1 and the second pressing portion P2 on the left side and the right side are pressed from above, whereby the adhesion part 2 is deformed and brought into close contact with the body surface B, the insides of the first concave portion 24 and the second concave portions 25 are brought into a negative pressure state, and the electrode member 1 adheres to the body surface B.

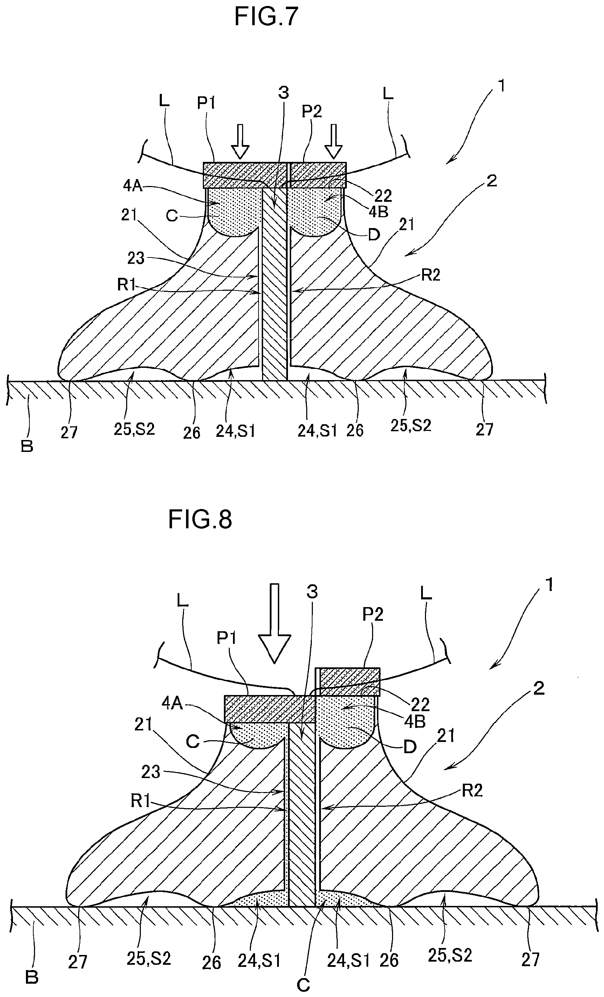

Subsequently, as illustrated in Fig. 7, the first pressing portion P1 and the second pressing portion P2 on the left side and the right side are pressed from above toward the body surface B side (second pressing). The adhesion part 2 is deformed by the second pressing to increase the negative pressures inside the first concave portion 24 and the second concave portions 25. At this time, the boundary portion 26 and the outer edge portion 27 are in contact with the body surface B, the volume of the internal space of the first concave portion 24 and the volume of the internal space of the second concave portion 25 decrease, and the first space S1 and the second space S2 sealed with the body surface B are formed.

Then, as illustrated in Fig. 8, only the first pressing portion P1 is pressed from above (third pressing). In the third pressing, only the first pressing portion P1 is pressed to cause the solution for dissolving sebum C to flow out into the first concave portion 24 through the first flow channel R1 and to cause the solution for dissolving sebum C to act on the body surface B in the first concave portion 24.

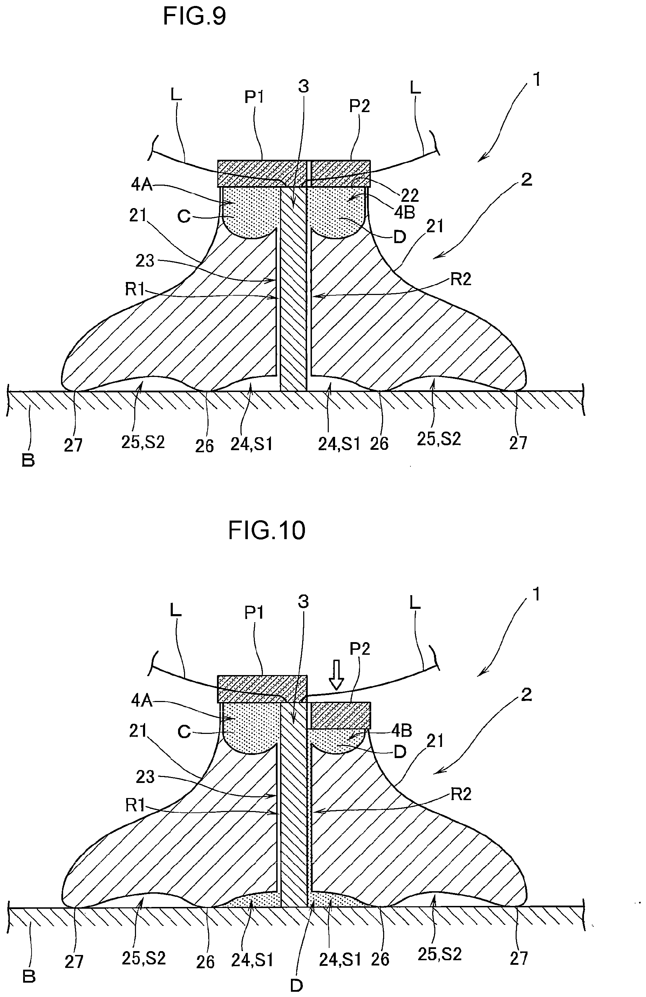

Thereafter, as illustrated in Fig. 9, most of the solution for dissolving sebum C is recovered in the first retention part 4A via the first flow channel R1 by utilizing the action of the spring of the first pressing portion P1 or the like over 30 to 60 minutes.

As illustrated in Fig. 10, only the second pressing portion P2 is pressed from above (fourth pressing). By the fourth pressing, the electrolyte gel D flows out into the first concave portion 24 through the second flow channel R2, and the inside of the first concave portion 24 can be filled with the electrolyte gel D. As a result, for example, during the PSG test, the electrolyte gel D can maintain good contact between the body surface B and the electrode 3, and can prevent contact failure caused by the shape, movement, or the like of the body surface B due to some body movements or the like.

After the inspection or after the treatment, when the electrode member 1 is removed, it is desirable to remove the solution for dissolving sebum C and the electrolyte gel D remaining on the body surface B with alcohol-soaked gauze or the like.

Also in the electrode member 1 of this embodiment, in the mode for massage, it is possible to stably stimulate the body surface B by applying a current from the electrode 3 to the body surface B, at a predetermined position, and it is possible to relax the user and promote falling asleep. In addition, in the mode for PSG test, it is possible to reliably capture a weak current (such as EEG) from the electrode 3 to the body surface B, and it is possible to improve the inspection accuracy while suppressing the cost and the inspection labor. Furthermore, the electrolyte gel D can maintain good contact between the body surface B and the electrode 3, and can prevent contact failure caused by the shape, movement, or the like of the body surface B due to some body movements or the like.

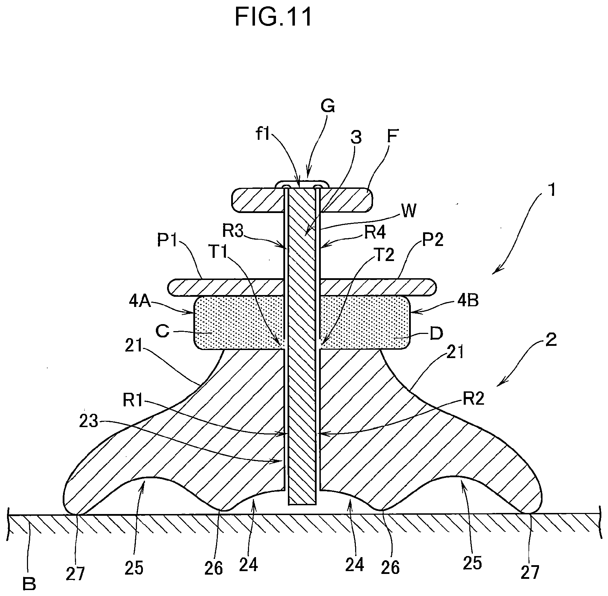

Next, a fourth embodiment of the electrode member of the present invention will be described with reference to Figs. 11 to 15. Portions common to those of the first embodiment and the third embodiment are denoted by the same reference signs, and description thereof will be partially omitted.

As illustrated in Fig. 11, the electrode member 1 of this embodiment includes a bag-shaped first retention part 4A and second retention part 4B on the upper side of the adhesion part 2. The first retention part 4A and the second retention part 4B are adjacent to each other with the electrode 3 interposed therebetween.

In the embodiment illustrated in Fig. 11, the first retention part 4A is provided on the left side in the drawing, and the solution for dissolving sebum C is retained in the first retention part 4A. In addition, the first retention part 4A communicates with the inside of the flow channel (first flow channel R1) via the first communication hole T1. The solution for dissolving sebum C is able to flow out from the first retention part 4A through the first flow channel R1 from one end side of the through hole 23.

In addition, the second retention part 4B is provided on the right side in the drawing, and the electrolyte gel D is retained in the second retention part 4B. In addition, the second retention part 4B communicates with the inside of the flow channel (second flow channel R2) via the second communication hole T2. The electrolyte gel D is able to flow out from one end side of the through hole 23 through the second flow channel R2. The component of the electrolyte gel D is not particularly limited, but it is preferable to contain a material that covers the narrowness of the contact area between the electrode 3 and the body surface B (prevents contact failure) and increases the conductivity.

The electrode member 1 includes a first pressing portion P1 on the upper side of the first retention part 4A and a second pressing portion P2 on the upper side of the second retention part 4B. The first pressing portion P1 and the second pressing portion P2 are divided and adjacent to each other, and can be operated independently.

The electrode member 1 includes a peripheral wall portion W around the upper side of the electrode 3, and a third flow channel R3 and a fourth flow channel R4 are formed between the electrode 3 and the peripheral wall portion W. The third flow channel R3 vertically faces the first flow channel R1 via the first communication hole T1, and the fourth flow channel R4 vertically faces the second flow channel R2 via the second communication hole T2.

The electrode member 1 includes an operation part F with a substantially disk shape, having a circular opening f1 at the center around the upper end of the electrode 3. The electrode 3 and the upper end side of the peripheral wall portion W are inserted into the circular opening f1 of the operation part F. Furthermore, a pressure adjustment mechanism G is provided so as to cover the upper end of the electrode 3 (the circular opening f1 of the operation part F). Air can be supplied, sucked, and the like via the pressure adjustment mechanism G, whereby the air pressure in the third flow channel R3 and the fourth flow channel R4 can be adjusted.

When the electrode member 1 of this embodiment is attached to the body surface B, the electrode member 1 is disposed at a predetermined position on the body surface B.

Next, the entire electrode member 1 is pushed from above toward the body surface B side (first pressing). By the first pressing, the adhesion part 2 is deformed and brought into close contact with the body surface B, the insides of the first concave portion 24 and the second concave portion 25 are brought into a negative pressure state, and the electrode member 1 adheres to the body surface B.

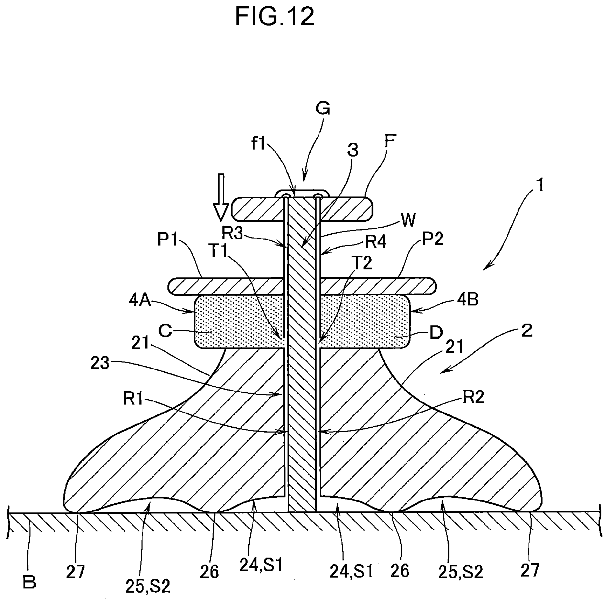

Subsequently, as illustrated in Fig. 12, the operation part F is pushed from above toward the body surface B side to cause the electrode 3 to come in contact with the body surface B (second pressing). The adhesion part 2 is further deformed by the second pressing to increase the negative pressures inside the first concave portion 24 and the second concave portion 25. At this time, the boundary portion 26 and the outer edge portion 27 are in contact with the body surface B, the volume of the internal space of the first concave portion 24 and the volume of the internal space of the second concave portion 25 decrease, and the first space S1 and the second space S2 sealed with the body surface B are formed. By the second pressing, the adhesion part 2 can be stably fixed in a state where the lower end portion of the electrode 3 comes in contact with the body surface B. In addition, at the time of the second pressing, the pressure in the first space S1 can be adjusted by the pressure adjustment mechanism G to increase the adhesion strength.

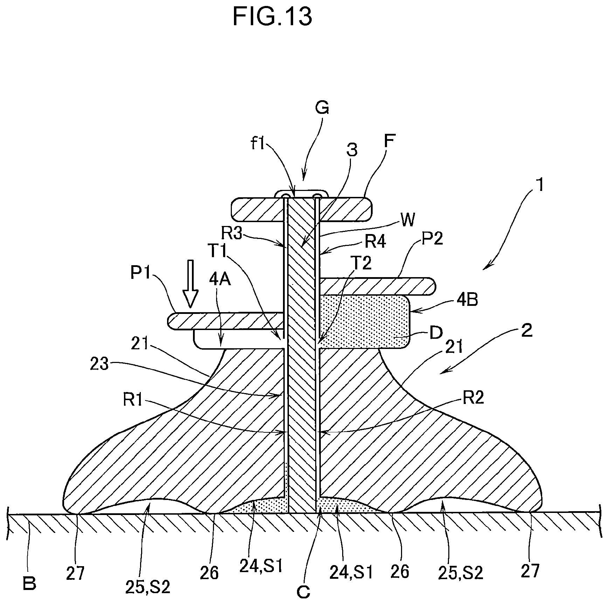

Then, as illustrated in Fig. 13, only the first pressing portion P1 is pressed downward (third pressing). The first retention part 4A is compressed by the third pressing, and the solution for dissolving sebum C in the first retention part 4A flows out to the first flow channel R1 through the first communication hole T1 and is supplied into the first concave portion 24, whereby the solution for dissolving sebum C acts on the body surface B.

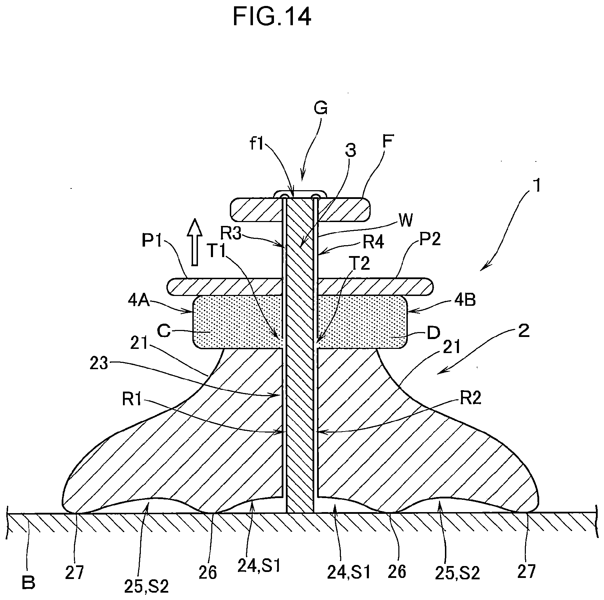

Thereafter, as illustrated in Fig. 14, most of the solution for dissolving sebum C is recovered in the first retention part 4A via the first flow channel R1 by utilizing the action of the spring of the first pressing portion P1 or the like over 30 to 60 minutes.

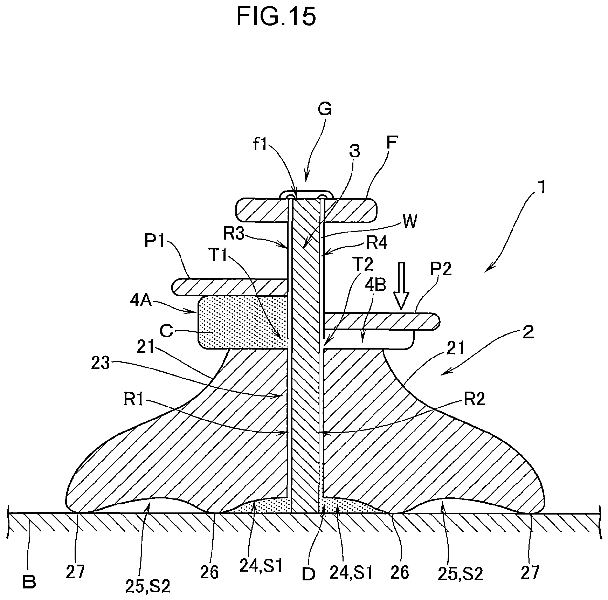

Next, as illustrated in Fig. 15, only the second pressing portion P2 is pressed downward (fourth pressing). The second retention part 4B is compressed by the fourth pressing, the electrolyte gel D in the second retention part 4B flows out to the second flow channel R2 through the second communication hole T2, and the first concave portion 24 is filled with the electrolyte gel D. As a result, for example, during the PSG test, the electrolyte gel D can maintain good contact between the body surface B and the electrode 3, and can prevent contact failure caused by the shape, movement, or the like of the body surface B due to some body movements or the like.

Note that the second pressing portion P2 may have a structure in which the state of being pressed by the fourth pressing is maintained, or may have a structure in which the second pressing portion P2 returns to the position before pressing similarly to the first pressing portion P1.

In addition, after the inspection or the treatment, for example, the adhesion part 2 can be removed from the body surface B by setting the insides of the first concave portion 24 and the second concave portion 25 to positive pressures using the pressure adjustment mechanism G or the like. After the electrode member 1 is removed, it is desirable to remove the solution for dissolving sebum C and the electrolyte gel D remaining on the body surface B with alcohol-soaked gauze or the like.

Also in the electrode member 1 of this embodiment, in the mode for massage, it is possible to stably stimulate the body surface B by applying a current from the electrode 3 to the body surface B, at a predetermined position, and it is possible to relax the user and promote falling asleep. In addition, in the mode for PSG test, it is possible to reliably capture a weak current (such as EEG) from the electrode 3 to the body surface B, and it is possible to improve the inspection accuracy while suppressing the cost and the inspection labor. Furthermore, the electrolyte gel D can maintain good contact between the body surface B and the electrode 3, and can prevent contact failure caused by the shape, movement, or the like of the body surface B due to some body movements or the like, so that a weak current can be captured.

As described above, the electrode member 1 of the present invention is an electrode member 1 to be attached to a body surface B of the user, and includes: an adhesion part 2 including a through hole 23 near a center and capable of adhering to a body surface B of the user;
an electrode 3 inserted into the through hole 23 of the adhesion part 2; and
a first retention part 4A configured to retain a solution for dissolving sebum C, in which
a flow channel R including a gap is formed between an inner peripheral surface 23A of the through hole 23 of the adhesion part 2 and the electrode 3, and
the solution for dissolving sebum C retained in the first retention part 4A is able to flow out from one end side of the through hole 23 through the flow channel R.

Next, an embodiment of a head orthosis, a body orthosis, and a wear set of the present invention will be described. Description of the above-described electrode member is omitted.

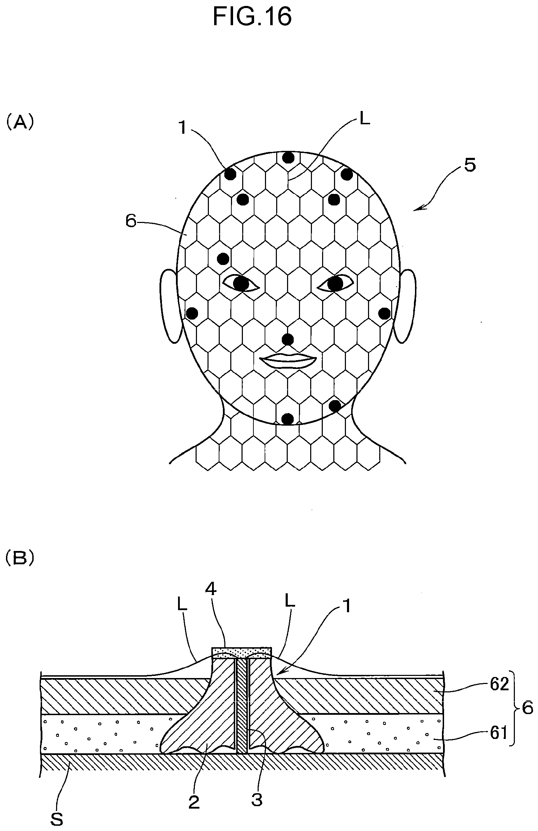

Fig. 16 includes a front view and a cross-sectional view illustrating an embodiment of the head orthosis of the present invention.

A head orthosis 5 of the present invention includes the electrode member 1 of the present invention described above and a head mounted member 6.

The head mounted member 6 preferably has a two-layer structure including a moisture absorbing layer 61 configured to come in contact with the head surface and an insulating layer 62 laminated on the moisture absorbing layer 61.

The material of the moisture absorbing layer 61 is not particularly limited, but is preferably a moisture absorbing material rich in stretchability, and thus, it is possible to reduce an adverse effect due to sweating.

In addition, the material of the insulating layer 62 is not particularly limited, but is preferably an insulating material rich in stretchability, durability, and heat retention. Since the disposing position of the electrode member 1 varies depending on the physique and the like for each individual, it is considered that the electrode member 1 has a material and a structure that can cope with the disposing position. The insulating layer 62 can block a current from flowing to the body surface side through the conductive wire L.

In addition, as the disposing position of the electrode member 1, a predetermined position defined in the international 10-20 system, a position of each pressure point, a desired position such as an auricle portion, an eyelid portion, and a lower jaw can be exemplified in accordance with the purpose. An opening penetrating the moisture absorbing layer 61 and the insulating layer 62 can be provided in accordance with the disposing position of the electrode member 1, and the electrode member 1 can be exposed from the surface of the head mounted member 6 through the opening.

Furthermore, in order to minimize the influence of electric leakage due to sweating in PSG that captures a weak current and massage that sends a normal current, the surface of the head mounted member 6 is preferably covered with a mesh body having a hygroscopic network structure in which units of substantially regular hexagons in a front view are continuous. The mesh body is preferably formed of a string-like insulator. With this mesh body, the electrode member 1 can be reliably fixed. In this case, for example, the conductive wire for the weak current and the normal current, extending from the electrode member 1, can be directly connected to the PSG measurement device, the controller, the massage apparatus main body, and the like.

In this mode, although not illustrated, a detachable fixture can be attached to the vicinity of the mesh-like structure separately from the electrode member. The conductive wire for weak current and the conductive wire for normal current extending from the electrode member can also be connected to a measurement device, a controller, and the like via the fixture. In this embodiment, the fixture may have a plurality of openings or the like for holding the conductive wire for weak current and the conductive wire for normal current.

Note that, in Figs. 16(A) and 16(B), a mesh-like structure including a conductive wire L described later is illustrated, but a similar structure can be adopted in a case of the above-described mesh body (string-like insulator).

On the other hand, as another mode, as illustrated in Figs. 16(A) and 16(B), a mode can be exemplified in which a conductive wire L connected to the electrode 3 of the electrode member 1 extends on the surface of the insulating layer 62, and a mesh-like structure in which units of substantially regular hexagons in front view are continuous is formed by the conductive wire L. The mesh-like structure of the conductive wire L can cover the entire head except for the eyes, mouth, and the like. The weak current from the surface of the head captured from the electrode 3 can be guided to the central portion, the controller, and the like through the mesh-like structure of the conductive wire L for each site and EEG.

In a case of the head orthosis of the present invention for PSG, since the string-like insulator or the conductive wire L forms the mesh-like structure together with the features of the electrode member described above, the disposing position of the electrode member 1 can be easily adjusted, so that the burden of preparation work for PSG test is reduced. Specifically, for example, the PSG test preparation work that conventionally takes 30 minutes to 40 minutes or more by one to two staff members can be completed by about 10 to 15 minutes by one staff member, and thus the PSG test can be carried out on several to 10 or more people on the same day, and a large cost cut can be realized. Since there is also a problem of a new coronavirus infection, the head orthosis 5 of the present invention should be disposable as long as the viral infection cannot be completely prevented by alcohol disinfection.

The size of the regular hexagonal unit structure formed by the string-like insulator (mesh body) or the conductive wire L is not particularly limited, and for example, can be appropriately designed according to the site and the intended use (PSG or massage). Specifically, for example, the length of a line segment passing through the center of the regular hexagon is preferably in the range of 5 mm to 10 cm.

In addition, in the head orthosis of the present invention, in a case of the mode for massage or in a case where the capture efficiency for PSG (for capturing weak current) is not sufficient by the mesh-like structure alone using the conductive wire, for example, it is preferable to directly connect the conductive wire for weak current and normal current extending from the electrode member 1 to the PSG measuring device, the controller, the massage apparatus main body, and the like.

In addition, the head orthosis 5 of the present invention can include, for example, an electrooculogram electrode, an oxygen saturation sensor, a system reference electrode, a geniomyoelectric electrode, an airflow sensor, and the like in the application of PSG test.

The electrooculogram electrode is an electrode for acquiring an electrooculogram (EOG) of the user. The electrooculogram electrode can be made of, for example, an elastic member impregnated with an electrolytic solution, and can be disposed, for example, near a temple of the user.

The oxygen saturation sensor is a sensor for acquiring a percutaneous oxygen saturation (SPO2) of a user. The oxygen saturation sensor may include a light emitting unit that irradiates the skin with light and a light receiving unit that measures reflected light, and may have a structure in which the oxygen saturation is determined from an absorbance difference of the reflected light due to the oxygen concentration. The oxygen saturation sensor can be disposed, for example, near the forehead of the user.

The system reference electrode is an electrode for acquiring a reference potential serving as a reference of an EEG electrode, an electrooculogram electrode, and a geniomyoelectric electrode described later. The system reference electrode can be made of, for example, an elastic member impregnated with an electrolytic solution.

The geniomyoelectric electrode is an electrode for acquiring a mental electromyograph (EMG). The geniomyoelectric electrode can be made of, for example, an elastic member impregnated with an electrolytic solution.

The airflow sensor is a sensor for acquiring a nasal-mouth respiration flow of the user. The airflow sensor may be a sensor that measures a differential pressure due to exhalation.

The same configuration as that of the above-described head orthosis of the present invention can be adopted for the body orthosis of the present invention (not illustrated). The description of a portion common to the above-described head orthosis of the present invention will be partially omitted.

A body orthosis of the present invention includes the electrode member of the present invention described above and a body mounted member.

The body mounted member covers the body (the entire body except the head) of the user, and is preferably brought into close contact with the body of the user. Specifically, for example, it is preferable to have the form of clothing similar to male ballet wear.

The body mounted member preferably has a two-layer structure including a moisture absorbing layer configured to come in contact with the body surface and an insulating layer laminated on the moisture absorbing layer. An opening penetrating the moisture absorbing layer and the insulating layer 62 can be provided in accordance with the disposing position of the electrode member, and the electrode member can be exposed from the surface of the body mounted member through the opening.

In the mode for PSG that captures a weak current and the mode for massage that sends a normal current, similarly to the mode of the head orthosis described above, the surface of the body mounted member is preferably covered with a mesh body having a mesh-like structure in which units of substantially regular hexagons in a front view are continuous. The mesh body is preferably a string-like insulator. With this mesh body, the electrode member can be reliably fixed.

In this case, for example, the conductive wire for the weak current and the normal current extending from the electrode member can be directly connected to the PSG measurement device, the controller, and the massage apparatus main body.

Also in this mode, a detachable fixture can be attached to the vicinity of the mesh-like structure separately from the electrode member. The conductive wire for weak current and the conductive wire for normal current extending from the electrode member can also be connected to a measurement device, a controller, and the like via the fixture.

The size of the regular hexagonal unit structure formed by the string-like insulator (mesh body) or the conductive wire is not particularly limited, and for example, can be appropriately designed according to the site and the intended use (PSG or massage). Specifically, for example, the length of a line segment passing through the center of the regular hexagon is preferably in the range of 5 mm to 10 cm.

On the other hand, as another mode, similarly to the mode of the head orthosis described above, a mode in which a mesh-like structure is formed by a conductive wire on the surface of the insulating layer, in the mesh-like structure, units of a substantially regular hexagon in a front view being continuous, can be exemplified. The mesh structure of the conductive wire can cover the entire body. The weak current from the body surface captured from the electrode of the electrode member can be guided to the central portion, the controller, and the like through the mesh-like structure of the conductive wire for each site or for each electrocardiogram, electromyogram, or the like. In addition, for example, also in a case of a normal current such as a massage application, the current from the power supply can be supplied to the body surface through the mesh-like structure of the conductive wire. Since the conductive wire forms the mesh-like structure, the disposing position of the electrode member can be easily adjusted. In addition, in this embodiment, an insulating layer of the body mounted member is not necessarily required, and the surface of the conductive wire can be coated with an insulating material.

In addition, in the body orthosis of the present invention, when the capture efficiency for massage and PSG (for capturing weak current) is not sufficient by the mesh-like structure alone using the conductive wire, for example, it is preferable to directly connect the conductive wire for weak current and normal current extending from the electrode member to the PSG measuring device, the controller, the massage apparatus main body, or the like.

The disposing position of the electrode member can be appropriately adjusted in accordance with the purpose, but for example, the body shape of the entire body can be grasped in detail and three-dimensionally by a 3D laser scanner for each individual user in advance, a map indicating the disposing position of the electrode member for each individual can be printed on the moisture absorbing layer on the inner side, and the electrode can be disposed by adjusting the position of the electrode.

In addition, the body orthosis can include, for example, an electrocardiographic sensor, a respiratory motion sensor, and the like.

The electrocardiographic sensor is a sensor for acquiring an electrocardiogram (ECG) of the user. The electrocardiographic sensor may be made of, for example, an elastic member impregnated with an electrolytic solution, and may have a structure electrically connectable to the chest of user.

The respiratory motion sensor is a sensor for acquiring respiratory motion of the user. As the respiratory motion sensor, for example, any sensor can be used such as a sensor that measures expansion and contraction of a belt fixed to the torso of the user.

Furthermore, in the body mounted member, the electrode member located on the back surface side of the user can be set to be adhered and fixed to the body surface of the user when a load is applied by the user.

The wear set (not illustrated) of the present invention includes the head orthosis and the body orthosis described above, and can be suitably used as a wear set for massage (preferably, a network structure of a regular hexagon fully made of an insulator is adopted) or PSG test (preferably, a network structure of a regular hexagon fully made of an insulator is adopted).

By using the electrode member of the present invention for a head orthosis or a body orthosis for massage or PSG test, it is possible to perform comfortable massage (acupuncture treatment alternative treatment) and reliable PSG test while suppressing the cost and the inspection labor.
In addition, the electrode member of the present invention and the head orthosis and the body orthosis including the electrode member may allow, for example, a local administration method (administration using low frequency or ultrasonic irradiation in combination) for increasing the local drug concentration to be used. In this case, for example, various drugs can be caused to flow out from the electrode member at an appropriate timing through the flow channel of the electrode member to act. In addition, in a mode in which the electrode member includes the first retention part and the second retention part, the drug is able to be retained in the first retention part and the second retention part. The type of the drug is not particularly limited, and examples of the drug for AGA (male alopecia) include a hair increasing agent (angiogenic agents, hormonal agents, etc.), and examples of the drug for skin lesion include an atopic therapeutic agent, a plaque psoriasis therapeutic agent, and an anticancer agent.

In the acupuncture treatment alternative treatment by the electrical stimulation to the head and the whole body, it is considered that the simultaneous or different energization to all the electrodes does not necessarily maximize the therapeutic effect for each individual user, for each complication, season, and weather, for each physical condition on that day, and the like. Therefore, it is possible to improve the effect to be obtained after the alternative treatment of acupuncture by changing the site and the number of electrodes to be energized, the intensity of the current to be energized, the time, the timing, the type of the conductive wire, the fixing position, and the like, and thereafter inputting and recording the effect of the treatment to a tablet or the like once every 1 to 2 weeks using a good sleep check table or the like, thereby causing the Al to perform feedback learning.

As the good sleep check table, for example, the treatment may be changed in a plurality of stages as follows.

| | |
|---|---|
| 8 Scores or less | In its present state |
| 9 to 14 Scores | Requires adjustment by equipment personnel |
| 15 to 20 Scores | Instruction such as change of prescribing instruction of attending physician is required. |
| 21 Scores or higher | Consultation with a sleep specialist or neuropsychiatry specialist is required |

Therefore, also in the treatment using the wear set of the present invention, the comfort of falling asleep and falling asleep again, and the like can be controlled with feedback by utilizing the Al on the basis of the information from the user on the basis of the possibility that the comfort differs for each individual according to the underlying disease and the physical condition and disease state at that time.

Therefore, before use of the wear set or the like of the present invention, it is possible to adjust, for initial and subsequent treatments, the treatment menu and contents such as the site and number of electrodes to be energized, and the intensity and duration of the energizing current, by Al according to the situation such as the AHI value at the time of sleep apnea syndrome diagnosis, by having a user input, to a tablet or the like, a cohabitation family structure (presence or absence of stress, nursing care status, etc.), height and weight (BMI), medical history (especially stroke and heart diseases such as arrhythmia), complications (nasal and oral related diseases such as chronic tonsillitis and sinusitis), current illness (hypertension, diabetes, heart diseases such as cardiac insufficiency, cerebrovascular disorder, asthma, pulmonary emphysema, liver disease, depression, and the like), family history, life history (sleeping habit (sleeping posture, mainly in a supine position, a right lateral position, and a left lateral position)), sleep duration (naps, second sleeps, etc.), bathing (time, temperature, sauna, etc.), coffee consumption (timing (daytime, evening or later), quantity, etc.), as well as smoking (past smoking history, current smoking and passive smoking), alcohol consumption (frequency, quantity, with/without blush), indulgence grocery items, etc., allergy history (pollinosis, asthma, etc.), normal blood pressure, pulse rate, and body temperature, recent symptoms, normal meal time, meal contents, presence or absence of eating between meals (contents), contents of work (presence or absence of stress (contents such as interpersonal relationship)), work time zone (including presence or absence of night shift, shift work, and the like), etc.

Furthermore, it is preferable to have the user input the physical condition of the day, the stress at work, the exercise time and content, the meal time and content, the presence or absence and quantity of alcohol consumption, the bathing time and content, and the like using a tablet or the like every day and during the time from dinner to before sleep, if possible, in a touch panel manner. In addition, after waking up, it is preferable to similarly have the user input the sleeping time and contents (including the number of times of waking up and the nighttime toilet frequency), the physical condition at the time of waking up, symptoms such as a good sleep feeling, a headache, and dryness, the state of the device and malfunction. As a result, it is possible to derive an optimum condition such as sleep for each individual and use the optimum condition for setting the sleep condition on the day of treatment.

In addition, better comfort can be pursued on a 1 to 4 week basis or by reviewing the conventional average, comparison with the previous week/period, and the like.

Furthermore, it is preferable to have the user input, to a tablet, a situation in which daytime sleepiness appears (for example: generally dozing off, at the time of sitting and reading a newspaper or a book, sitting and watching TV, sitting and sitting quietly in a conference or a movie theater or a theater, sitting on a car driven by another person continuously for one hour, lying down and resting in the afternoon, sitting and talking with a person, sitting quietly after lunch without drinking alcohol, or stopping for a few minutes due to traffic jam while driving by oneself, etc.) as an item related to subjective symptoms.

In addition, it is preferable that the user also input whether or not the user has the following symptoms.

Frequently snoring, waking up with difficulty in breathing, waking up with a desire to urinate twice or more at night, feeling a decrease in memory and concentration, feeling a decrease in memory power and concentration, feeling lethargy and being unable to recover from tiredness, being pointed out by family members to stop breathing during sleep and apnea for several minutes, having shallow or frequently waking up at night, having heaviness of head or headache at wake-up or during the morning, feeling chronic sleep deprivation without a deep sleep feeling at wake-up, drinking alcohol daily instead of taking sleeping medication, and the like.

As described above, smart good sleep is realized by utilizing AI, and big data regarding good sleep and re-falling asleep is utilized for research and development, and more comfortable falling asleep and re-falling asleep are permanently pursued.

The electrode member, the head orthosis, the body orthosis, and the wear set of the present invention are not limited to the above embodiments.

### Reference Signs List

- 1: Electrode member
- 2: Adhesion part
- 23: Through hole
- 24: First concave portion
- 25: Second concave portion
- 3: Electrode
- 4A: First retention part
- 4B: Second retention part
- 5: Head orthosis
- 6: Head mounted member
- 61: Moisture absorbing layer
- 62: Insulating layer
- C: Solution for dissolving sebum
- D: Electrolyte gel
- L: Conductive wire
- R: Flow channel
- R1: First flow channel
- R2: Second flow channel
- R3: Third flow channel
- R4: Fourth flow channel

## Claims

1. An electrode member (1) to be attached to a body surface (B) of a user, comprising:
an adhesion part (2) including a through hole (23) near a center and capable of adhering to a body surface of a user;
an electrode (3) inserted into the through hole (23) of the adhesion part (2); and
a first retention part (4A) configured to retain a solution (C) for dissolving sebum, wherein
a flow channel (R) including a gap is formed between an inner peripheral surface (23A) of the through hole (23) of the adhesion part (2) and the electrode (3), and
the solution for dissolving sebum retained in the first retention part (4A) is able to flow out from one end side of the through hole (23) through the flow channel (R).

2. The electrode member (1) according to claim 1, wherein
the adhesion part (2) includes
a first concave portion (24) formed around the one end side of the through hole (23) and connected to the flow channel (R), and
the solution for dissolving sebum (C) is sent from the first retention part (4A) to the first concave portion (24) through the flow channel (R).

3. The electrode member (1) according to claim 2, wherein the adhesion part (2) includes a second concave portion (25) formed around the first concave portion (24) and adheres to a body surface of a user via the second concave portion (25).

4. The electrode member (1) according to any one of claims 1 to 3, wherein the first retention part (4A) is provided on another end side of the through hole (23).

5. The electrode member (1) according to any one of claims 1 to 4, further comprising a second retention part (4B) configured to retain an electrolyte gel (D), wherein the electrolyte gel is able to flow out from the one end side of the through hole (23) from the second retention part (4B) through the flow channel (R).

6. The electrode member (1) according to any one of claims 1 to 5, wherein the electrode member (1) is configured to be used for massage or for PSG test.

7. A head orthosis (5) comprising: the electrode member (1) according to any one of claims 1 to 6; and a head mounted member (6).

8. The head orthosis (5) according to claim 7, wherein the head mounted member (6) includes a moisture absorbing layer (61) configured to come in contact with a head surface, and an insulating layer (62) laminated on the moisture absorbing layer.

9. The head orthosis (5) according to claim 8 comprising a conductive wire (L) connected to an electrode (3) of the electrode member (1), wherein
in the conductive wire (L), a mesh structure in which units of a substantially regular hexagon in a front view are continuous is formed, and a surface of the head mounted member (6) on a side of the insulating layer (62) is covered with the conductive wire (L).

10. A body orthosis comprising: the electrode member (1) according to any one of claims 1 to 6; and a body mounted member.

11. The body orthosis according to claim 10, wherein the body mounted member includes a moisture absorbing layer configured to come in contact with a body surface, and an insulating layer laminated on the moisture absorbing layer.

12. The body orthosis according to claim 11 comprising a conductive wire connected to an electrode (3) of the electrode member (1), wherein
in the conductive wire, a mesh structure in which units of a substantially regular hexagon in a front view are continuous is formed, and a surface of the head mounted member on a side of the insulating layer is covered with the conductive wire.

13. A wear set comprising the head orthosis (5) according to any of claims 7 to 9 and the body orthosis according to any of claims 10 to 12.

## Patentansprüche

1. Elektrodenelement (1) zum Anbringen an einer Körperoberfläche (B) eines Benutzers, umfassend:
einen Haftteil (2), der ein Durchgangsloch (23) in der Nähe einer Mitte einschließt und der an einer Körperoberfläche eines Benutzers haften kann;
eine Elektrode (3), die in das Durchgangsloch (23) des Haftteils (2) eingeführt ist;
und einen ersten Halteteil (4A), der zum Halten einer Lösung (C) zum Auflösen von Talg konfiguriert ist, wobei
ein Strömungskanal (R) einschließlich eines Spaltes zwischen einer Innenumfangsfläche (23A) des Durchgangslochs (23) des Haftteils (2) und der Elektrode (3) ausgebildet ist und die in dem ersten Halteteil (4A) gehaltene Lösung zum Auflösen von Talg aus einer Endseite des Durchgangslochs (23) durch den Strömungskanal (R) heraus strömen kann.

2. Elektrodenelement (1) nach Anspruch 1, wobei
der Haftteil (2) Folgendes einschließt
einen ersten konkaven Abschnitt (24), der um die eine Endseite des Durchgangslochs (23) herum ausgebildet und mit dem Strömungskanal (R) verbunden ist, und
wobei die Lösung zum Auflösen von Talg (C) von dem ersten Halteteil (4A) durch den Strömungskanal (R) zu dem ersten konkaven Abschnitt (24) gesendet wird.

3. Elektrodenelement (1) nach Anspruch 2, wobei der Haftteil (2) einen zweiten konkaven Abschnitt (25) einschließt, der um den ersten konkaven Abschnitt (24) herum ausgebildet ist und über den zweiten konkaven Abschnitt (25) an einer Körperoberfläche eines Benutzers haftet.

4. Elektrodenelement (1) nach einem der Ansprüche 1 bis 3, wobei der erste Halteteil (4A) an einer anderen Endseite des Durchgangslochs (23) bereitgestellt ist.

5. Elektrodenelement (1) nach einem der Ansprüche 1 bis 4, weiter umfassend einen zweiten Halteteil (4B), der so konfiguriert ist, dass er ein Elektrolytgel (D) hält, wobei das Elektrolytgel aus der einen Endseite des Durchgangslochs (23) aus dem zweiten Halteteil (4B) durch den Strömungskanal (R) heraus strömen kann.

6. Elektrodenelement (1) nach einem der Ansprüche 1 bis 5, wobei das Elektrodenelement (1) so konfiguriert ist, dass es für Massagen oder für PSG-Tests verwendet werden kann.

7. Kopforthese (5), umfassend: das Elektrodenelement (1) nach einem der Ansprüche 1 bis 6; und ein am Kopf montiertes Element (6).

8. Kopforthese (5) nach Anspruch 7, wobei das am Kopf montierte Element (6) eine feuchtigkeitsabsorbierende Schicht (61), die so konfiguriert ist, dass sie mit einer Kopfoberfläche in Kontakt kommt, und eine auf die feuchtigkeitsabsorbierende Schicht laminierte Isolierschicht (62) einschließt.

9. Kopforthese (5) nach Anspruch 8, umfassend einen leitfähigen Draht (L), der mit einer Elektrode (3) des Elektrodenelements (1) verbunden ist, wobei
in dem leitfähigen Draht (L) eine Netzstruktur ausgebildet ist, in der Einheiten eines im Wesentlichen regelmäßigen Sechsecks in einer Vorderansicht kontinuierlich sind, und eine Oberfläche des am Kopf montierten Elements (6) auf einer Seite der Isolierschicht (62) mit dem leitfähigen Draht (L) bedeckt ist.

10. Körperorthese, umfassend: das Elektrodenelement (1) nach einem der Ansprüche 1 bis 6; und ein am Körper montiertes Element.

11. Körperorthese nach Anspruch 10, wobei das am Körper montierte Element eine feuchtigkeitsabsorbierende Schicht, die so konfiguriert ist, dass sie mit einer Körperoberfläche in Kontakt kommt, und eine auf die feuchtigkeitsabsorbierende Schicht laminierte Isolierschicht einschließt.

12. Körperorthese nach Anspruch 11, umfassend einen leitfähigen Draht, der mit einer Elektrode (3) des Elektrodenelements (1) verbunden ist, wobei
in dem leitfähigen Draht eine Netzstruktur ausgebildet ist, in der Einheiten eines im Wesentlichen regelmäßigen Sechsecks in einer Vorderansicht kontinuierlich sind, und eine Oberfläche des am Kopf montierten Elements auf einer Seite der Isolierschicht mit dem leitfähigen Draht bedeckt ist.

13. Trage-Set, das die Kopforthese (5) nach einem der Ansprüche 7 bis 9 und die Körperorthese nach einem der Ansprüche 10 bis 12 umfasst.

## Revendications

1. Organe (1) d'électrode à fixer à une surface corporelle (B) d'un utilisateur, comprenant :
une partie d'adhérence (2) incluant un trou traversant (23) près d'un centre et apte à adhérer à une surface corporelle d'un utilisateur ;
une électrode (3) insérée dans le trou traversant (23) de la partie d'adhérence (2) ; et une première partie de rétention (4A) configurée pour retenir une solution (C) pour dissoudre le sébum, dans lequel
un canal d'écoulement (R) incluant un espace est formé entre une surface périphérique interne (23A) du trou traversant (23) de la partie d'adhérence (2) et l'électrode (3), et la solution pour dissoudre le sébum retenue dans la première partie de rétention (4A) est apte à s'écouler à partir d'un côté d'extrémité du trou traversant (23) à travers le canal d'écoulement (R).

2. Organe (1) d'électrode selon la revendication 1, dans lequel la partie d'adhérence (2) inclut
une première portion concave (24) formée autour du un côté d'extrémité du trou traversant (23) et reliée au canal d'écoulement (R), et
la solution (C) pour dissoudre le sébum est envoyée de la première partie de rétention (4A) à la première portion concave (24) à travers le canal d'écoulement (R).

3. Organe (1) d'électrode selon la revendication 2, dans lequel la partie d'adhérence (2) inclut une seconde portion concave (25) formée autour de la première portion concave (24) et adhère à une surface corporelle d'un utilisateur via la seconde portion concave (25).

4. Organe (1) d'électrode selon l'une quelconque des revendications 1 à 3, dans lequel la première partie de rétention (4A) est prévue sur un autre côté d'extrémité du trou traversant (23).

5. Organe (1) d'électrode selon l'une quelconque des revendications 1 à 4, comprenant en outre une seconde partie de rétention (4B) configurée pour retenir un gel d'électrolyte (D), dans lequel le gel d'électrolyte est apte à s'écouler à partir de l'un côté d'extrémité du trou traversant (23) à partir de la seconde partie de rétention (4B) à travers le canal d'écoulement (R).

6. Organe (1) d'électrode selon l'une quelconque des revendications 1 à 5, dans lequel l'organe (1) d'électrode est configuré pour être utilisé pour un massage ou pour un test PSG.

7. Orthèse (5) de tête comprenant : l'organe (1) d'électrode selon l'une quelconque des revendications 1 à 6 ; et un organe (6) monté sur tête.

8. Orthèse (5) de tête selon la revendication 7, dans laquelle l'organe (6) monté sur tête inclut une couche (61) absorbant l'humidité configurée pour entrer en contact avec une surface de tête, et une couche isolante (62) stratifiée sur la couche absorbant l'humidité.

9. Orthèse (5) de tête selon la revendication 8 comprenant un fil conducteur (L) relié à une électrode (3) de l'organe (1) d'électrode, dans laquelle
dans le fil conducteur (L), une structure maillée dans laquelle des unités d'un hexagone sensiblement régulier en vue de face sont continues est formée, et une surface de l'organe (6) monté sur tête sur un côté de la couche isolante (62) est recouverte par le fil conducteur (L).

10. Orthèse de corps comprenant : l'organe (1) d'électrode selon l'une quelconque des revendications 1 à 6 ; et un organe monté sur corps.

11. Orthèse de corps selon la revendication 10, dans laquelle l'organe monté sur corps inclut une couche absorbant l'humidité configurée pour entrer en contact avec une surface corporelle, et une couche isolante stratifiée sur la couche absorbant l'humidité.

12. Orthèse de corps selon la revendication 11 comprenant un fil conducteur relié à une électrode (3) de l'organe (1) d'électrode, dans laquelle
dans le fil conducteur, une structure maillée dans laquelle des unités d'un hexagone sensiblement régulier en vue de face sont continues est formée, et une surface de l'organe monté sur tête sur un côté de la couche isolante est recouverte par le fil conducteur.

13. Ensemble à porter comprenant l'orthèse (5) de tête selon l'une quelconque des revendications 7 à 9 et l'orthèse de corps selon l'une quelconque des revendications 10 à 12.
